# EUROPÄISCHE PATENTSCHRIFT

(11) **EP 2 364 655 B1**
(45) Veröffentlichungstag und Bekanntmachung des Hinweises auf die Patenterteilung: **08.05.2013**
(21) Anmeldenummer: 11157250.9
(22) Anmeldetag: 08.03.2011
(51) Int. Cl.: A61B 17/42, A61B 17/29

(54) **Medizinisches Instrument**
Medical instrument
Instrument médical

(30) Priorität: 10.03.2010 DE 102010010947
(43) Veröffentlichungstag der Anmeldung: 14.09.2011
(73) Patentinhaber: Karl Storz GmbH & Co. KG, 78532 Tuttlingen (DE)
(72) Erfinder: Hahn, Martin, 78532, Tuttlingen (DE); Doll, Frank, 78532, Tuttlingen (DE)
(74) Vertreter: Fugmann, Winfried

(56) Entgegenhaltungen:
- EP-A1- 1 695 669
- EP-A2- 0 606 531
- DE-U1-202004 019 868
- US-A- 5 382 252

## Beschreibung

Die Erfindung liegt auf dem Gebiet der Medizintechnik und betrifft nach ihrer Gattung ein medizinisches Instrument gemäß dem Oberbegriff von Anspruch 1.

Gattungsgemäße medizinische Instrumente werden im Handel in großer Zahl vertrieben und sind in der einschlägigen Patentliteratur bereits vielfach beschrieben worden. Lediglich beispielhaft sei in diesem Zusammenhang auf die deutsche Gebrauchsmusterschrift DE 202004019868 U1 verwiesen. Zwei nach DE 2020040168 U1 beispielhaft hergestellte medizinische Instrumente sind der von der Anmelderin im Handel vertriebene Uterus-Manipulator Modell Clermont-Ferrand (Geräte-Nummer 26168 D) bzw. der Uterus-Manipulator nach Tintara (Geräte-Nummer 26168 TN).

Demgegenüber besteht die Aufgabe der vorliegenden Erfindung darin, ein gattungsgemäßes medizinisches Instrument in vorteilhafter Weise weiterzubilden.

Diese und weitere Aufgaben werden nach dem Vorschlag der Erfindung durch ein medizinisches Instrument mit den Merkmalen des unabhängigen Anspruchs gelöst. Vorteilhafte Ausgestaltungen der Erfindung sind durch die Merkmale der Unteransprüche angegeben.

Gemäß vorliegender Erfindung umfasst ein medizinisches Instrument einen proximalen Instrumententeil und einen distalen Instrumententeil, die über einen langgestreckten, beispielsweise rohrförmigen Schaft miteinander verbunden sind. Das distale Instrumententeil weist ein in Bezug auf die Längsrichtung bzw. Längsachse des Schafts um eine Schwenkachse schwenkbar gelagertes Schwenkelement auf, das durch ein translatorisch bewegbares Betätigungselement, beispielsweise eine Druck-/Zugstange, verschwenkbar ist.

Das medizinische Instrument zeichnet sich in wesentlicher Weise dadurch aus, dass das Schwenkelement durch wenigstens ein Koppelelement mit dem Betätigungselement gekoppelt ist, wobei das wenigstens eine Koppelelement mit dem Schwenkelement über eine erste Gelenkverbindung und mit dem Betätigungselement über eine zweite Gelenkverbindung jeweils gelenkig verbunden ist. Die erste Gelenkverbindung des Koppelelements verbindet das Koppelelement außerhalb der Schwenkachse des Schwenkelements mit diesem, so dass über das Koppelelement ein Drehmoment auf das Schwenkelement übertragen und das Schwenkelement durch das Koppelelement verschwenkt werden kann. Die erste Gelenkverbindung folgt somit der Schwenkbewegung des Schwenkteils. Die zweite Gelenkverbindung des Koppelements ist durch wenigstens eine vom distalen Instrumententeil geformte nicht-lineare Führung (mittelbar oder unmittelbar) geführt, derart, dass das Schwenkelement sowohl in der einen Schwenkrichtung als auch in der anderen Schwenkrichtung verschwenkbar ist. Zudem ist das Betätigungselement zur Führung durch die nicht-lineare Führung geeignet ausgebildet, so dass es der durch die nicht-lineare Führung vorgegebenen Bewegungsbahn folgen kann. Das Betätigungselement kann zu diesem Zweck beispielsweise einen distalen Endabschnitt aufweisen, der an dem übrigen Abschnitt des Betätigungselements schwenkbar angelenkt ist. Alternativ hierzu ist es beispielsweise auch möglich, dass das Betätigungselement biegbar ausgebildet ist.

Das erfindungsgemäße medizinische Instrument ermöglicht somit in vorteilhafter Weise ein Verschwenken des Schwenkelements sowohl in der einen Schwenkrichtung als auch in der anderen Schwenkrichtung. Dies wird in technisch einfacher Weise durch das wenigstens eine Koppelelement zur Übertragung eines Drehmoments auf das Schwenkelement erreicht, welches sowohl am Schwenkelement als auch am Betätigungselement angelenkt ist, wobei die gelenkige Verbindung zwischen Koppelelement und Betätigungselement durch eine nicht-lineare Führung in geeigneter Weise mittelbar oder unmittelbar geführt ist.

Bei einer vorteilhaften Ausgestaltung des medizinischen Instruments ist die nicht-lineare Führung winkelig ausgebildet und umfasst einen entlang der durch den Schaft definierten Längsachse sich erstreckenden ersten Abschnitt, im Weiteren als "Längsabschnitt" bezeichnet, und einen schräg zur Längsachse sich erstreckenden, zweiten Abschnitt, im Weiteren als "Schrägabschnitt" bezeichnet. Der Schrägabschnitt ist hierbei so zur Längsachse angestellt, dass über die erste Gelenkverbindung zwischen Koppelelement und Schwenkelement ein Drehmoment auf das Schwenkelement übertragen werden kann.

Wie oben bereits ausgeführt, kann in dem erfindungsgemäßen medizinischen Instrument die zweite Gelenkverbindung des Koppelelements von der nicht-linearen Führung unmittelbar (direkt) geführt sein. In diesem Fall kann es besonders vorteilhaft sein, wenn die nicht-lineare Führung in Form einer Kulissenführung ausgebildet ist, wobei die zweite Gelenkverbindung des Koppelelements zu diesem Zweck wenigstens ein als Kulissenzapfen in die Kulissenführung greifendes Gelenkglied aufweisen kann. Durch diese Maßnahme kann eine unmittelbare Führung der zweiten Gelenkverbindung in besonders einfacher Weise realisiert werden.

Alternativ hierzu ist es auch möglich, dass die zweite Gelenkverbindung des Koppelelements von der nicht-linearen Führung lediglich mittelbar (indirekt) geführt wird. Insbesondere kann in diesem Fall beispielsweise das Betätigungselement von der nicht-linearen Führung geführt sein, um eine mittelbare Führung der zweiten Gelenkverbindung durch die nicht-lineare Führung zu erzielen.

Bei einer weiteren vorteilhaften Ausgestaltung des medizinischen Instruments ist das wenigstens eine Koppelteil bogenförmig ausgebildet. Durch diese Maßnahme kann erreicht werden, dass das Schwenkelement über einen relativ großen Schwenkbereich verschwenkt wird, wobei das bogenförmige Koppelteil über eine Schwenkwelle des Schwenkelements geführt werden kann, ohne dass die Gefahr einer Kollision zwischen Koppelelement und Schwenkwelle besteht.

Bei einer weiteren vorteilhaften Ausgestaltung des medizinischen Instruments ist das Schwenkelement, ausgehend von einer Mittenlage, um wenigstens 90° in der einen Schwenkrichtung und wenigstens 90° in der anderen Schwenkrichtung verschwenkbar, wobei durch diese Maßnahme ein für die praktische Anwendung besonders großer Schwenkbereich realisiert ist.

Die Erfindung wird nun anhand eines Ausführungsbeispiels näher erläutert, wobei Bezug auf die beigefügten Zeichnungen genommen wird. Es zeigen:
- Fig. 1: in einer perspektivischen Seitenansicht ein Ausführungsbeispiel des erfindungsgemäßen Instruments;
- Fig. 2: in einer perspektivischen Ansicht von schräg oben den distalen Instrumententeil des Instruments von Fig. 1;
- Fig. 3: in einer teiltransparenten Seitenansicht den distalen Instrumententeil des Instruments von Fig. 1;
- Fig. 4: in einer teiltransparenten Aufsicht den distalen Instrumententeil des Instruments von Fig. 1;
- Fig. 5: in einer teiltransparenten Seitenansicht den distalen Instrumententeil des Instruments von Fig. 1 mit einem in der einen Drehrichtung verschwenkten Schwenkteil;
- Fig. 6: in einer teiltransparenten Seitenansicht den distalen Endabschnitt des Instruments von Fig. 1 mit einem in der anderen Drehrichtung verschwenkten Schwenkteil.

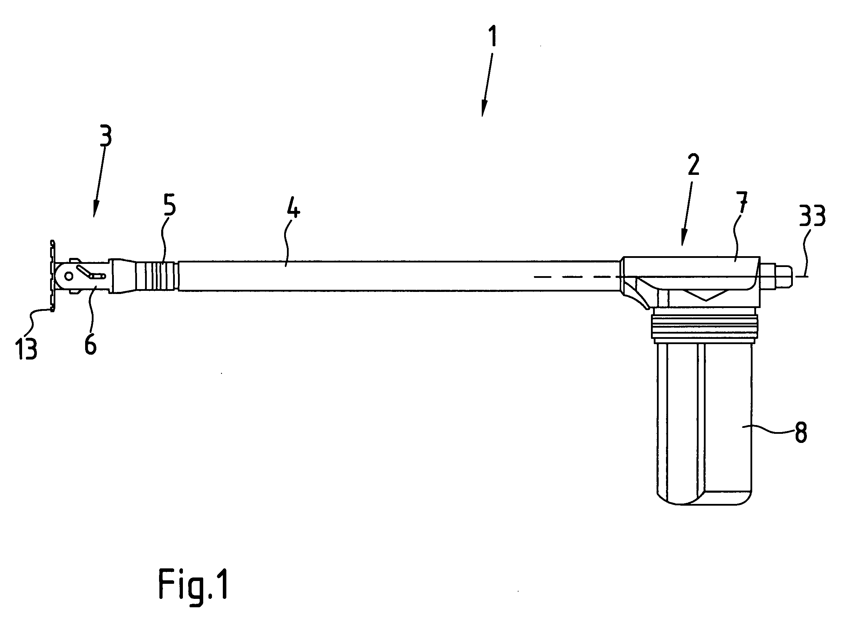

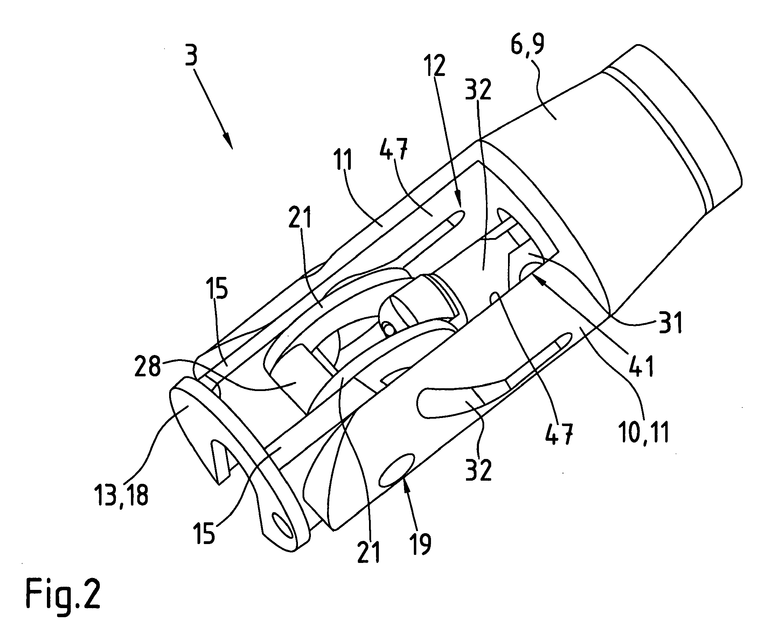

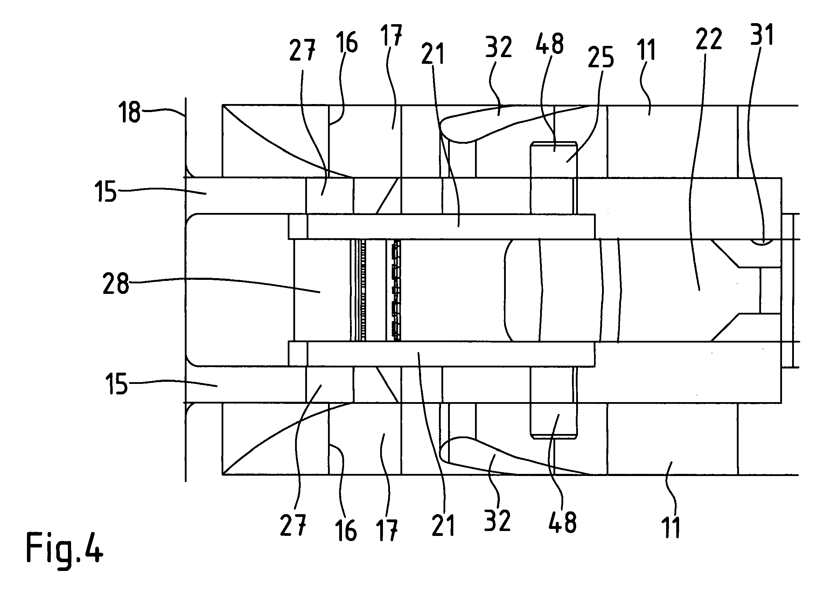

Unter Bezugnahme auf Fig. 1 bis Fig. 6 wird nun ein insgesamt mit der Bezugszahl 1 bezeichnetes Instrument beschrieben. Hierbei gemachte Lage- und Richtungsangaben, wie "oben" und "unten", beziehen sich zum Zwecke einer einfacheren Beschreibung lediglich auf die in den Figuren dargestellte Ausrichtung des Instruments 1. Es versteht sich von selbst, dass das Instrument 1 anders ausgerichtet sein kann, so dass diese Angaben in keiner Weise als einschränkend aufgefasst werden sollen.

Das in Verbindung mit den Figuren erläuterte Instrument 1 dient insbesondere dazu, ein Organ in seiner Lage zu verändern und/oder zu fixieren, beispielsweise um laparaskopische Untersuchungen oder Behandlungen vorzunehmen.

Das Instrument 1 umfasst einen proximalen Instrumententeil 2 und einen distalen Instrumententeil 3, die über einen in Fig. 1 verkürzt dargestellten rohrförmigen Schaft 4 miteinander verbunden sind. Durch die Rohrachse des Schafts 4 ist eine Längsrichtung bzw. Längsachse 33 des Instruments 1 definiert.

Der proximale Instrumententeil 2 ist mit dem proximalen Ende des Schafts 4 mit einem mit dem Handgriff 8 zum Halten des Instruments 1 fest verbundenen Aufsatzteil 7 verbunden. Das Aufsatzteil 7 ist durch eine herkömmliche beispielsweise lösbare Verbindung wie eine Schraub- oder Rastverbindung am Handgriff 8 befestigt. Zu diesem Zweck kann das Aufsatzteil 7 beispielsweise mit einem (nicht dargestellten) Rohrstutzen versehen sein, auf den der Handgriff 8 aufgeschoben ist. In entsprechender Weise ist der Schaft 4 in herkömmlicher Weise am Aufsatzteil 7 beispielsweise lösbar angebracht. Der Handgriff 8 umfasst ein mit dem Aufsatzteil 7 starr verbundenes erstes Griffteil 49 sowie ein zweites Griffteil 50, das an dem Schwenkgelenk 51 relativ zum ersten Griffteil 49 verschwenkt werden kann.

Der distale Instrumententeil 3 weist einen über ein Anschlussteil 5 am distalen Ende des Schafts 4 montierten Gelenkkopf 6 auf, wobei der Gelenkkopf 6 am Anschlussteil 5 über eine herkömmliche lösbare Verbindung, wie beispielsweise eine Schraub- oder Rastverbindung, befestigt ist. Das Anschlussteil 5 ist am Schaft 4 in herkömmlicher Weise lösbar oder auch nicht lösbar angebracht.

Der Gelenkkopf 6 ist mit einem zum distalen Ende des Instruments 1 hin sich radial verbreiternden Basisabschnitt 9 versehen, an den sich distal ein Gelenkabschnitt 10 mit zwei zueinander parallelen Gelenkgabeln 11 anschließt. Die beiden Gelenkgabeln 11 erstrecken sich entlang der Längsachse 33 und formen zwischen sich eine in etwa quaderförmige Ausnehmung 12 mit einander zugewandten planen Innenflächen 47. Sie dienen zum Anlenken eines Schwenkteils 13 am Gelenkkopf 6.

Das Schwenkteil 13 ist mit zwei Schwenkarmen 15 versehen, die von den beiden Gelenkgabeln 11 umgriffen werden. Zur gelenkigen Verbindung mit den Gelenkgabeln 11 weisen die beiden Schwenkarme 15 nicht näher dargestellte Durchgangsbohrungen auf, die von einem ersten Gelenkstift 17 durchsetzt werden, der in einander ge-genüberliegenden ersten Lagerbohrungen 16 der Gelenkgabeln 11 aufgenommen ist. In einer ersten Variante ist der erste Gelenkstift 17 drehbar in den ersten Lagerbohrungen 16 gelagert, während die Schwenkarme 15 mit dem ersten Gelenkstift 17 drehfest verbunden sind. In einer zweiten Variante ist der erste Gelenkstift 17 in den ersten Lagerbohrungen 16 drehfest gelagert, während die Schwenkarme 15 mit dem ersten Gelenkstift 17 drehbar verbunden sind. Alternativ wäre es gleichermaßen möglich, dass anstelle des ersten Gelenkstifts 17 jeweils ein Gelenkzapfen zwischen Schwenkarm 15 und benachbarter Gelenkgabel 11 vorgesehen ist, durch den der Schwenkarm 15 relativ zur Gelenkgabel 11 verschwenkt werden kann.

Demnach ist das Schwenkteil 13 über ein insgesamt mit der Bezugszahl 19 bezeichnetes Schwenkgelenk, im Folgenden als "Schwenkteilgelenk" bezeichnet, am Gelenkkopf 6 schwenkbar gelagert. Durch Lage und Richtung des ersten Gelenkstifts 17 ist eine erste Schwenkachse 29 definiert, die senkrecht zur Längsachse 33 ist.

Die beiden Schwenkarme 15 des Schwenkteils 13 sind durch eine senkrecht zur Längsachse 33 angeordnete Trägerplatte 18 miteinander verbunden. Die Trägerplatte 18, welche sich außerhalb der durch die beiden Gelenkgabeln 11 gebildeten Ausnehmung 12 befindet, dient zur lösbaren Montage einer entsprechend den spezifischen Erfordernissen für die Manipulation des Organs benötigten Einrichtung (nicht dargestellt), deren räumliche Lage durch Verschwenken des Schwenkteils 13 veränderbar ist. Denkbar wäre auch, eine solche Einrichtung ohne Trägerplatte 18 direkt mit den beiden Schwenkarmen 15 zu verbinden.

Das Schwenkteil 13 ist mit einer translatorisch bewegbaren Druck-/Zugstange 20 (in der Beschreibungseinleitung als "Betätigungselement" bezeichnet) gekoppelt. Die Druck-/Zugstange 20 ist in einem Hohlraum des rohrförmigen Schafts 4 aufgenommen und erstreckt sich proximal durch das Aufsatzteil 7 hindurch bis zum Handgriff 8 und distal bis in den Gelenkkopf 6 hinein. Sie kann durch einen manuell betätigbaren Mechanismus zum Erzeugen einer translatorischen Bewegung hin und her bewegt werden, so dass sie in Abhängigkeit der jeweiligen Bewegungsrichtung als Zug- oder Druckstange wirkt. In dem in den Fig. 1 veranschaulichten Ausführungsbeispiel ist die Druck-/Zugstange 20 zu diesem Zweck mit einem Befestigungsabschnitt 52 des zweiten Griffteils 50 fest verbunden (nicht dargestellt), so dass sie durch Betätigen des Handgriffs 8 translatorisch verschoben werden kann. Zur Verringerung der Reibung ist sie innerhalb einer Hülse 30 aus einem reibungsarmen Material, beispielsweise Teflon, aufgenommen. Derartige Mechanismen zum Bewegen der Druck-/Zugstange 20 sind dem Fachmann sowohl aus der einschlägigen Patentliteratur als auch durch im Handel verfügbare Instrumente wohlbekannt.

Das Schwenkteil 13 kann durch die entlang der Längsachse 33 bidirektional bewegbare Druck-/Zugstange 20 in verschiedene Winkelpositionen relativ zum Schaft 4 bzw. Längsachse 33 verschwenkt werden. Zu diesem Zweck ist eine Wirkkopplung vorgesehen, die in Längsachse 33 sich erstreckende, bogenförmige Koppelteile 21 umfasst, die sowohl mit der Druck-/Zugstange 20 als auch mit den beiden Schwenkarmen 15 des Schwenkteils 13 jeweils gelenkig verbunden sind. Insbesondere sind die proximalen Enden der Koppelteile 21 an einen distalen Endabschnitt der Druck-/Zugstange 20, nämlich einen mit dem übrigen Stangenabschnitt 31 gelenkig verbundenen Stangenkopf 22, angelenkt. Für eine gelenkige Verbindung zwischen dem Stangenkopf 22 und den beiden den Stangenkopf 22 gabelförmig umgreifenden Koppelteilen 21, ist der Stangenkopf 22 mit einer ersten Durchgangsbohrung 23 versehen, die fluchtend zu zweiten Durchgangsbohrungen 24 der beiden Koppelteile 21 angeordnet ist. Die ersten und zweiten Durchgangsbohrungen 23, 24 werden von einem zweiten Gelenkstift 25 durchsetzt, der mit seinen beiden Enden an den Gelenkgabeln 11 gelagert ist. Zu Lagerung des zweiten Gelenkstifts 25 sind die beiden Gelenkgabeln 11 mit als Durchbrechungen ausgebildeten Kulissen 32 versehen, in denen die Enden des zweiten Gelenkstifts 25 aufgenommen sind. In einer ersten Variante sind entweder die beiden Koppelteile 21 oder der Stangenkopf 22 drehfest mit dem zweiten Gelenkstift 25 verbunden, während der zweite Gelenkstift 25 drehbar und verschiebbar in den Kulissen 32 gelagert ist. In einer zweiten Variante sind die Koppelteile 21 und der Stangenkopf 22 drehbar mit dem zweiten Gelenkstift 25 verbunden, während der zweite Gelenkstift 25 drehfest, aber verschiebbar in den Kulissen 32 gelagert ist.

Demnach sind die beiden Koppelteile 21 über ein insgesamt mit der Bezugszahl 36 bezeichnetes Gelenk, im Weiteren als "proximales Koppelteilgelenk" bezeichnet, am Stangenkopf 22 schwenkbar gelagert. Durch Lage und Ausrichtung des zweiten Gelenkstifts 25 ist eine zweite Schwenkachse 37 definiert, die senkrecht zur Längsachse 33 bzw. parallel zur ersten Schwenkachse 29 des Schwenkteils 13 verläuft.

An ihren anderen Enden sind die beiden Koppelteile 21 jeweils mit einem Schwenkarm 15 gelenkig verbunden, wobei die beiden Schwenkarme 15 die Koppelteile 21 gabelförmig umgreifen. Altermativ ist natürlich auch eine Ausführung möglich, bei der die Koppelteile 21 die beiden Schwenkarme 15 umgreifen oder aber ein Koppelteil 21 bezüglich der Längsachse 33 außerhalb, das andere innerhalb seines zugehörigen bzw. angesteuerten Schwenkarms 15 angebracht ist. Zur gelenkigen Verbindung der Koppelteile 21 mit den Schwenkarmen 15 sind die Koppelteile 21 jeweils mit zweiten Lagerbohrungen 26 versehen, in denen ein am benachbarten Schwenkarm 15 angeformter Gelenkzapfen 27 drehbar aufgenommen ist. Alternativ wäre es gleichermaßen möglich, dass die Gelenkzapfen 27 an den Koppelteilen 21 angeformt und in entsprechenden Lagerbohrungen der Schwenkarme 15 drehbar aufgenommen sind.

Demnach sind die beiden Koppelteile 21 über ein insgesamt mit der Bezugszahl 39 bezeichnetes Gelenk, im Weiteren als "distales Koppelteilgelenk" bezeichnet, an den Schwenkarmen 15 schwenkbar gelagert. Durch Lage und Ausrichtung der beiden Gelenkzapfen 27 ist eine dritte Schwenkachse 40 definiert, die senkrecht zur Längsachse 33 bzw. parallel zur zweiten Schwenkachse 37 verläuft.

Die beiden Koppelteile 21 greifen außerhalb der ersten Schwenkachse 29 an den beiden Schwenkarmen 15 an, so dass zwischen dem Angriffspunkt des distalen Koppelteilgelenks 39 und der ersten Schwenkachse 29 ein Hebel gebildet ist, über den ein Drehmoment zum Verschwenken des Schwenkteils 13 übertragen werden kann. Zwischen den beiden Koppelteilen 21 ist im Bereich des distalen Koppelteilgelenks 39 ein Distanzstück 28 vorgesehen, durch das die beiden Koppelteile 21 gegeneinander versteift werden.

Bei einer translatorischen Bewegung der Druck-/Zugstange 20 dienen die Kulissen 32 als Kulissenführung für den zweiten Gelenkstift 25 des proximalen Koppelteilgelenks 36, wobei die beiden Enden des zweiten Gelenkstifts 25 als Kulissenzapfen 48 in die Kulissen 32 greifen. Im vorliegenden Ausführungsbeispiel sind die Kulissen 32 als Durchbrechungen der Gelenkgabeln 11 ausgebildet. Alternativ wäre es gleichermaßen möglich, die Kulissen 32 beispielsweise als nutartige Einformungen der einander zugewandten Innenflächen 47 der Gelenkgabeln 11 auszubilden.

Die beiden Kulissen 32 haben in einer Ebene, die von der Längsachse 33 und einer Senkrechten zu einer jeden der Schwenkachsen 29, 37, 40 aufgespannt wird, eine winkelige Form. Sie setzen sich von proximal nach distal aus einem entlang der Längsachse 33 sich erstreckenden (linearen) Längsabschnitt 34 und einem schräg zur Längsachse 33 sich erstreckenden (linearen) Schrägabschnitt 35 mit in etwa gleicher Länge zusammen. In den Figuren ist der Schrägabschnitt 35 der Kulissen 32 gegenüber der Längsachse 33 so angestellt, dass beim Verschieben der Druck-/Zugstange 20 durch das distale Koppelteilgelenk 39 ein Drehmoment auf das Schwenkteil 13 ausgeübt wird. Bezogen auf eine Ebene, die durch die Längsachse 33 und die erste Schwenkachse 29 des Schwenkteilgelenks 19 aufgespannt ist, erstreckt sich der Schrägabschnitt 35 zu diesem Zweck von proximal nach distal in jenen Halbraum hinein, in dem sich das distale Koppelteilgelenk 39 befindet. In dem in den Figuren gezeigten Ausführungsbeispiel ist der Schrägabschnitt 35 gegenüber der Längsachse 33 nach oben abgewinkelt, wobei ein Winkel zwischen dem Schrägabschnitt 35 und der Längsachse 33 beispielsweise ca. 30° beträgt. Jedoch kann ein hiervon abweichender Winkel, je nach Art und Auslegung der Gelenkkopplung zwischen dem Schwenkteil 13 und der Druck-/Zugstange 20 vorgesehen sein.

Damit der Stangenkopf 22 der durch die beiden Kulissen 32 auferlegten, nicht-geraden Bewegungsbahn folgen kann, ist der Stangenkopf 22 durch ein insgesamt mit der Bezugszahl 41 bezeichnetes Gelenk, im Weiteren als "Stangenkopfgelenk" bezeichnet, am restlichen Stangenabschnitt 31 angelenkt. Zu diesem Zweck ist das distale Ende des Stangenabschnitts 31 mit dritten Durchgangsbohrungen 42 versehen, welche von einem dritten Gelenkstift 43 durchsetzt werden. Durch den dritten Gelenkstift 43 ist eine vierte Schwenkachse 44 definiert, die senkrecht zur Längsachse 33 gerichtet ist, so dass der Gelenkkopf 6 dem Verlauf des Schrägabschnitts 35 folgen kann.

Das proximale Koppelteilgelenk 36 wird somit beim translatorischen Verschieben der Druck-/Zugstange 20 durch die Kulissen 32 unmittelbar geführt, um das Schwenkteil 13 zu verschwenken, wobei ein vom Schrägabschnitt 35 geformter distaler Endanschlag 45 und ein vom Längsabschnitt 34 geformter proximaler Endanschlag 46 jeweils Endpunkte für die bidirektionale translatorische Bewegung der Druck-/Zugstange 20 bzw. ihren distalen Stangenkopf 22 vorgeben.

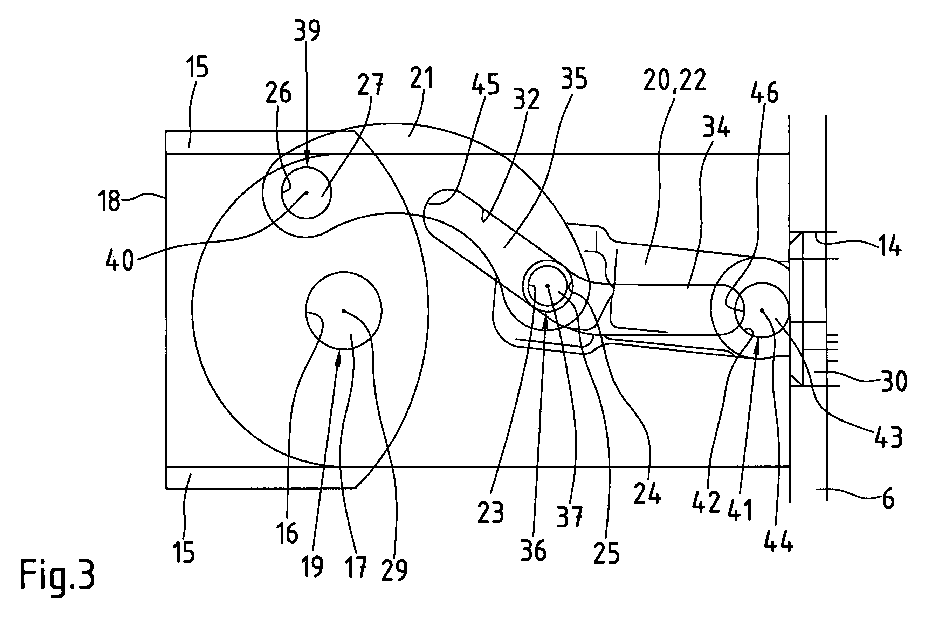

In Fig. 3 ist eine Situation dargestellt, bei der sich das proximale Koppelteilgelenk 36 in etwa in Mittenlage bezüglich des bidirektionalen Bewegungshubs der Druck-/Zugstange 20 befindet. Der Mittenlage der Druck-/Zugstange 20 ist hier beispielsweise eine Winkelposition des Schwenkteils 13 parallel zur Längsachse 33 zugeordnet. Das Schwenkteil 13 weist somit keine Auslenkung in Bezug auf den Schaft 4 auf (Winkelposition 0°). Alternativ hierzu, wäre jedoch auch denkbar, dass das Schwenkteil 13 bei Mittenlage der Druck-/Zugstange 20 in Bezug auf den Schaft 4 eine von 0° verschiedene Winkelposition hat.

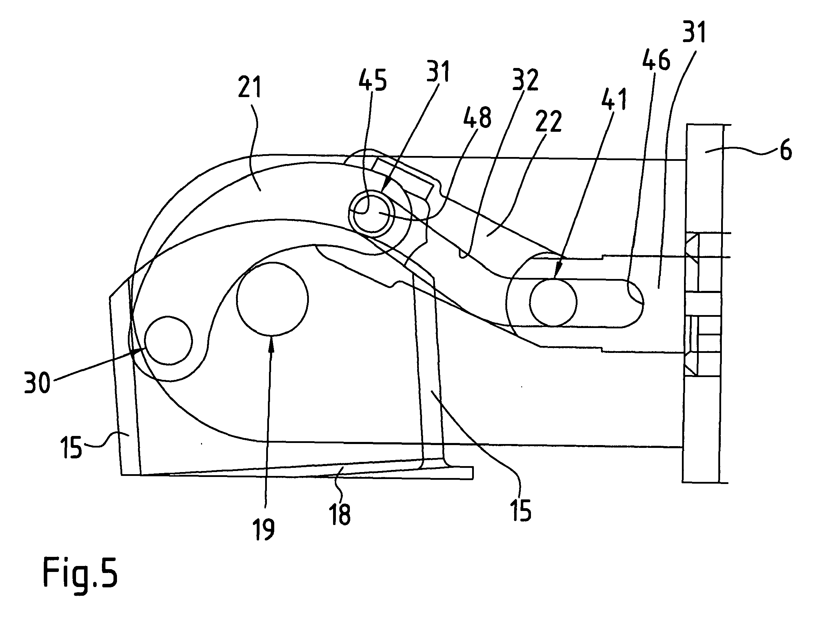

Wird die Druck-/Zugstange 20 aus ihrer Mittenlage translatorisch nach distal verschoben, hat dies zur Folge, dass das Schwenkteil 13 durch die beiden Koppelteile 21 in der einen Drehrichtung (in den Figuren entgegen dem Gegenuhrzeigersinn) verschwenkt wird, bis die Kulissenzapfen 48 des zweiten Gelenkstifts 25 des proximalen Koppelteilgelenks 36 zur Anlage gegen den distalen Endanschlag 45 der Kulissen 32 gelangen. In Fig. 5 ist eine Situation dargestellt, bei der die Kulissenzapfen 48 dem distalen Endanschlag 45 anliegen, entsprechend einem maximalen Bewegungshub der Druck-/Zugstange 20 nach distal. Bezogen auf den Schaft 4 weist das Schwenkteil 13 eine (hier beispielsweise mit positiven Gradzahlen bezeichnete) Winkelposition von ca. +120° auf.

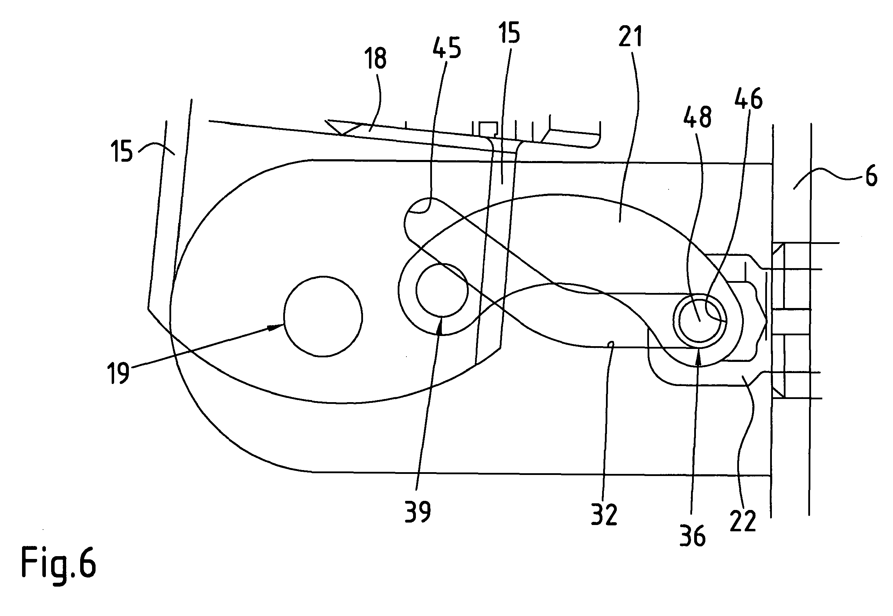

Wird die Druck-/Zugstange 20 aus ihrer Mittenlage in der entgegen gesetzten Bewegungsrichtung translatorisch nach proximal verschoben, wird das Schwenkteil 13 durch die beiden Koppelteile 21 in der entgegen gesetzten Drehrichtung, d.h. in den Figuren im Uhrzeigersinn, verschwenkt, bis die Kulissenzapfen 48 des zweiten Gelenkstifts 25 zur Anlage gegen den proximalen Endanschlag 46 der Kulissen 32 gelangt. In Fig. 6 ist eine Situation dargestellt, bei der die Kulissenzapfen 48 dem proximalen Endanschlag 46 anliegen, entsprechend einem maximalen Bewegungshub der Druck-/Zugstange 20 nach proximal. Bezogen auf den Schaft 4 weist das Schwenkteil 13 in dieser Stellung eine (hier beispielsweise mit positiven Gradzahlen bezeichnete) Auslenkung von ca. -120° auf.

Demnach kann das Schwenkteil 13, ausgehend von einer Mittenlage, in der sich die beiden Schwenkarme 15 parallel zum Schaft 4 erstrecken, durch Hin- und Herbewegen der Druck-/Zugstange 20 sowohl in der einen Drehrichtung als auch in der anderen Drehrichtung verschwenkt werden.

Durch die bogenförmige Ausgestaltung der Koppelteile 21 kann das Schwenkteil 13 in vorteilhafter Weise über einen relativ weiten Schwenkbereich verschwenkt werden, ohne dass diese mit dem die beiden Gelenkgabeln 11 miteinander verbindenden, ersten Gelenkstift 17 des Schwenkteilgelenks 19 kollidieren. Jedoch wäre es gleichermaßen auch möglich, anstelle bogenförmiger Koppelteile 21 anders geformte Koppelteile vorzusehen. Beispielsweise könnten gerade Koppelteile eingesetzt werden, wobei in diesem Fall gegebenenfalls der erste Gelenkstift 17 des Schwenkteilgelenks 19 durch jeweilige Gelenkzapfen zwischen den Gelenkgabeln 11 und den Schwenkarmen 15 zu ersetzen wäre. Gleichermaßen könnte anstelle des am Stangenabschnitt 31 mittels des Stangenkopfgelenks 41 angelenkten Stangenkopfs 22 eine biegbare Druck-/Zugstange 20 vorgesehen sein, deren distaler Endabschnitt in der Lage ist, der durch die Kulissen 32 auferlegten Bewegungsbahn zu folgen.

Durch das erfindungsgemäße Instrument 1 werden die herkömmlichen gattungsgemäßen Instrumente in vorteilhafter Weise weitergebildet, wobei das Schwenkteil 13 über eine technisch einfach zu realisierenden Kopplung mittels einer translatorisch bewegbaren Druck-/Zugstange 20 in beiden Schwenkrichtungen verschwenkbar ist.

### Bezugszeichenliste

- 1: Instrument
- 2: proximaler Instrumententeil
- 3: distaler Instrumententeil
- 4: Schaft
- 5: Anschlussteil
- 6: Gelenkkopf
- 7: Aufsatzteil
- 8: Handgriff
- 9: Basisabschnitt
- 10: Gelenkabschnitt
- 11: Gelenkgabel
- 12: Ausnehmung
- 13: Schwenkteil
- 14: Axialbohrung
- 15: Schwenkarm
- 16: erste Lagerbohrung
- 17: erster Gelenkstift
- 18: Trägerplatte
- 19: Schwenkteilgelenk
- 20: Druck-/Zugstange
- 21: Koppelteil
- 22: Stangenkopf
- 23: erste Durchgangsbohrung
- 24: zweite Durchgangsbohrung
- 25: zweiter Gelenkstift
- 26: zweite Lagerbohrung
- 27: Gelenkzapfen
- 28: Distanzstück
- 29: erste Schwenkachse
- 30: Hülse
- 31: Stangenabschnitt
- 32: Kulisse
- 33: Längsachse
- 34: Längsabschnitt
- 35: Schrägabschnitt
- 36: proximales Koppelteilgelenk
- 37: zweite Schwenkachse
- 39: distales Koppelteilgelenk
- 40: dritte Schwenkachse
- 41: Stangenkopfgelenk
- 42: dritte Durchgangsbohrung
- 43: dritter Gelenkstift
- 44: vierte Schwenkachse
- 45: distaler Endanschlag
- 46: proximaler Endanschlag
- 47: Innenfläche
- 48: Kulissenzapfen
- 49: erstes Griffteil
- 50: zweites Griffteil
- 51: Schwenkgelenk
- 52: Befestigungsabschnitt

## Patentansprüche

1. Medizinisches Instrument (1) mit einem proximalen Instrumententeil (2) und einem distalen Instrumententeil (3), die über einen langgestreckten Schaft (4) miteinander verbunden sind, wobei das distale Instrumententeil (3) ein in Bezug auf den Schaft (4) schwenkbar gelagertes Schwenkelement (13) umfasst, das durch ein translatorisch bewegbares Betätigungselement (20) verschwenkbar ist, **dadurch gekennzeichnet, dass**
- das Schwenkelement (13) durch wenigstens ein Koppelelement (21) mit dem Betätigungselement (20) gekoppelt ist, wobei das Koppelelement (21) mit dem Schwenkelement (13) über eine erste Gelenkverbindung (39) und mit dem Betätigungselement (20) über eine zweite Gelenkverbindung (36) gelenkig verbunden ist,
- die zweite Gelenkverbindung (36) des Koppelements (21) durch wenigstens eine vom distalen Instrumententeil (3) geformte nicht-lineare Führung (32) so geführt ist, dass das Schwenkelement (13) sowohl in der einen Schwenkrichtung als auch in der anderen Schwenkrichtung verschwenkbar ist, und
- das Betätigungselement (20) zur Führung durch die nicht-lineare Führung (32) geeignet ausgebildet ist.

2. Medizinisches Instrument (1) nach Anspruch 1, **dadurch gekennzeichnet, dass** die nicht-lineare Führung (32) winkelig ausgebildet ist, mit einem in Längsachse (33) des Schafts (4) sich erstreckenden Längsabschnitt (34) und einem schräg zur Längsachse (33) sich erstreckenden Schrägabschnitt (35).

3. Medizinisches Instrument (1) nach einem der Ansprüche 1 bis 2, **dadurch gekennzeichnet, dass** die zweite Gelenkverbindung (36) des Koppelelements (21) von der nichtlinearen Führung (32) unmittelbar geführt ist.

4. Medizinisches Instrument (1) nach Anspruch 3, **dadurch gekennzeichnet, dass** die nicht-lineare Führung (32) als Kulissenführung ausgebildet ist.

5. Medizinisches Instrument (1) nach Anspruch 4, **dadurch gekennzeichnet, dass** die zweite Gelenkverbindung (36) des Koppelelements (21) wenigstens ein in die Kulissenführung (32) greifendes Gelenkglied (25) umfasst.

6. Medizinisches Instrument (1) nach einem der Ansprüche 1 bis 2, **dadurch gekennzeichnet, dass** die zweite Gelenkverbindung (36) des Koppelelements (21) von der nichtlinearen Führung (32) mittelbar geführt ist.

7. Medizinisches Instrument (1) nach einem der Ansprüche 1 bis 6, **dadurch gekennzeichnet, dass** das Betätigungselement (20) einen distalen Endabschnitt (22) aufweist, der an einen übrigen Abschnitt (31) des Betätigungselements (20) schwenkbar angelenkt ist.

8. Medizinisches Instrument (1) nach einem der Ansprüche 1 bis 6, **dadurch gekennzeichnet, dass** das Betätigungselement (20) biegbar ausgebildet ist.

9. Medizinisches Instrument (1) nach einem der Ansprüche 1 bis 8, **gekennzeichnet durch** wenigstens ein bogenförmiges Koppelelement (21).

10. Medizinisches Instrument (1) nach einem der Ansprüche 1 bis 9, **dadurch gekennzeichnet, dass** der distale Instrumententeil (3) einen gegabelten Abschnitt (10) zur Anlenkung des Schwenkelements (13) umfasst.

11. Medizinisches Instrument (1) nach einem der Ansprüche 1 bis 10, **dadurch gekennzeichnet, dass** das Schwenkelement (13), ausgehend von einer Mittenlage, um wenigstens 90° in der einen Schwenkrichtung und wenigstens 90° in der anderen Schwenkrichtung verschwenkbar ist.

## Claims

1. Medical Instrument (1) with a proximal instrument part (2) and a distal instrument part (3) that are connected to each other by an elongate shaft (4), the distal instrument part (3) comprising a pivot element (13) which is mounted pivotably with respect to the shaft (4) and can be pivoted by an actuation element (20) movable in translation, **characterised in that**
- the pivot element (13) is coupled to the actuation clement (20) by at least one coupling element (21), wherein the coupling element (21) is connected in an articulated manner to the pivot element (13) via a first hinge connection (39) and to the actuation element (20) via a second hinge connection (36),
- the second hinge connection (36) of the coupling element (21) is guided by at least one non-linear guide (32) formed by the distal instrument part (3), such that the pivot element (13) is pivotable both in one direction of pivoting and in the other direction of pivoting, and
- the actuation element (20) is suitably designed to be guided by the non-linear guide (32).

2. Medical instrument (1) according to Claim 1, **characterized in that** the non-linear guide (32) has an angled configuration, with a longitudinal portion (34) extending in the longitudinal axis (33) of the shaft (4), and with an oblique portion (35) extending obliquely with respect to the longitudinal axis (33).

3. Medical instrument (1) according to either of Claims 1 and 2, **characterized in that** the second hinge connection (36) of the coupling element (21) is guided directly by the non-linear guide (32).

4. Medical instrument (1) according to Claim 3, **characterized in that** the non-linear guide (32) is designed as a slotted guide.

5. Medical instrument (1) according to Claim 4, **characterized in that** the second hinge connection (36) of the coupling element (21) comprises at least one hinge member (25) engaging in the slotted guide (32).

6. Medical instrument (1) according to either of Claims 1 and 2, **characterized in that** the second hinge connection (36) of the coupling element (21) is guided indirectly by the non-linear guide (32).

7. Medical instrument (1) according to one of Claims 1 to 6, **characterized in that** the actuation element (20) has a distal end portion (22), which is articulated pivotably on another portion (31) of the actuation element (20).

8. Medical instrument (1) according to one of Claims 1 to 6, **characterized in that** the actuation element (20) is designed to be flexible.

9. Medical instrument (1) according to one of Claims 1 to 8, **characterized by** at least one arc-shaped coupling element (21).

10. Medical instrument (1) according to one of Claims 1 to 9, **characterized in that** the distal instrument part (3) comprises a forked portion (10) for the articulation of the pivot element (13).

11. Medical instrument (1) according to one of Claims 1 to 10, **characterized in that** the pivot element (13), starting from a middle position, is pivotable through at least 90° in one direction of pivoting and at least 90° in the other direction of pivoting.

## Revendications

1. Instrument médical (1) comprenant une partie d'instrument proximale (2) et une partie d'instrument distale (3), qui sont connectées l'une à l'autre par le biais d'une tige (4) allongée, la partie d'instrument distale (3) comprenant un élément pivotant (13) monté de manière pivotante par rapport à la tige (4), qui peut être pivoté par un élément d'actionnement (20) déplaçable en translation, **caractérisé en ce que**
- l'élément pivotant (13) est accouplé par au moins un élément d'accouplement (21) à l'élément d'actionnement (20), l'élément d'accouplement (21) étant connecté de manière articulée à l'élément pivotant (13) par le biais d'une première liaison articulée (39) et à l'élément d'actionnement (20) par le biais d'une deuxième liaison articulée (36),
- la deuxième liaison articulée (36) de l'élément d'accouplement (21) est guidée par au moins un guide non linéaire (32) formé par la partie d'instrument distale (3) de telle sorte que l'élément pivotant (13) puisse pivoter à la fois dans un sens de pivotement et dans l'autre, et
- l'élément d'actionnement (20) est réalisé de manière appropriée pour le guidage par le guide non linéaire (32).

2. Instrument médical (1) selon la revendication 1, **caractérisé en ce que** le guide non linéaire (32) est réalisé sous forme coudée avec une portion longitudinale (34) s'étendant dans l'axe longitudinal (33) de la tige (4) et une portion oblique (35) s'étendant obliquement par rapport à l'axe longitudinal (33).

3. Instrument médical (1) selon l'une quelconque des revendications 1 à 2, **caractérisé en ce que** la deuxième liaison articulée (36) de l'élément d'accouplement (21) est guidée directement par le guide non linéaire (32).

4. Instrument médical (1) selon la revendication 3, **caractérisé en ce que** le guide non linéaire (32) est réalisé sous forme de guide à coulisse.

5. Instrument médical (1) selon la revendication 4, **caractérisé en ce que** la deuxième liaison articulée (36) de l'élément d'accouplement (21) comprend au moins un organe articulé (25) venant en prise dans le guide à coulisse (32).

6. Instrument médical (1) selon l'une quelconque des revendications 1 à 2, **caractérisé en ce que** la deuxième liaison articulée (36) de l'élément d'accouplement (21) est guidée de manière indirecte par le guide non linéaire (32).

7. Instrument médical (1) selon l'une quelconque des revendications 1 à 6, **caractérisé en ce que** l'élément d'actionnement (20) présente une portion d'extrémité distale (22), qui est articulée de manière pivotante à une autre portion (31) de l'élément d'actionnement (20).

8. Instrument médical (1) selon l'une quelconque des revendications 1 à 6, **caractérisé en ce que** l'élément d'actionnement (20) est réalisé de manière flexible.

9. Instrument médical (1) selon l'une quelconque des revendications 1 à 8, **caractérisé par** au moins un élément d'accouplement (21) de forme courbe.

10. Instrument médical (1) selon l'une quelconque des revendications 1 à 9, **caractérisé en ce que** la partie d'instrument distale (3) comprend une portion fourchue (10) pour l'articulation de l'élément pivotant (13).

11. Instrument médical (1) selon l'une quelconque des revendications 1 à 10, **caractérisé en ce que** l'élément pivotant (13), à partir d'une position centrale, peut être pivoté d'au moins 90° dans un sens de pivotement et d'au moins 90° dans l' autre sens de pivotement.
